(19) Europäisches Patentamt
European Patent Office
Office européen des brevets

(11) Publication number: 0 334 041
A2

# (12) EUROPEAN PATENT APPLICATION

(21) Application number: 89103093.4

(22) Date of filing: 22.02.89

(51) Int. Cl.4: A01N 57/20 , A01N 43/40 , C07D 213/64 , //(A01N57/20, 43:40),(A01N43/40,43:40)

(30) Priority: 23.02.88 JP 40389/88
24.02.88 JP 41751/88

(43) Date of publication of application:
27.09.89 Bulletin 89/39

(84) Designated Contracting States:
DE ES FR GB IT

(71) Applicant: MITSUBISHI PETROCHEMICAL CO., LTD.
5-2, 2-chome, Marunouchi
Chiyoda-ku Tokyo 100(JP)

(72) Inventor: Jikihara, Kazuo
1307-10, Onabake-machi
Ushiku-shi Ibaraki-ken(JP)
Inventor: Morishige, Jiro
11-6-306, Chuo 1-chome Ami-machi
Inashiki-gun Ibaraki-ken(JP)
Inventor: Endo, Keiji
11-5-203, Chuo 1-chome Ami-machi
Inashiki-gun Ibaraki-ken(JP)
Inventor: Goh, Atsushi
1977-7, Kosaka-machi
Ushiku-shi Ibaraki-ken(JP)
Inventor: Imada, Satoshi
11-4-302, Chuo 1-chome Ami-machi
Inashiki-gun Ibaraki-ken(JP)

(74) Representative: Kraus, Walter, Dr. et al
Patentanwälte Kraus, Weisert & Partner
Thomas-Wimmer-Ring 15
D-8000 München 22(DE)

## (54) Herbicidal composition.

(57) A herbicidal composition comprising
(A) a 2-nitro-5-(substituted pyridyloxy)benzohydroximic acid derivative represented by the following formula

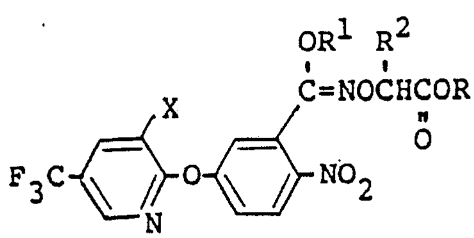

(I)

wherein R and $R^1$, independently from each other, represent a lower alkyl group, $R^2$ represents a hydrogen atom or a lower alkyl group, and X represents a hydrogen or halogen atom, and

EP 0 334 041 A2

(B) at least one compound selected from the group consisting of N-(phosphonomethyl)glycine, ammonium(3-amino-3-carboxypropyl)methylphosphinate, (2-amino-4-methylphosphinobutyryl)alanylalanine and 1,1′-dimethyl-4,4′-bipyridinium dichloride.

This invention relates to a novel herbicidal composition, and more specifically, to a herbicidal composition comprising a combination of a certain novel 2-nitro-5-(substituted pyridyloxy)benzohydroximic acid and a certain known herbicidally active compound as active ingredients, and a method of controlling weeds by using the composition.

N-(phosphonomethyl)glycine ("glyphosate"--see Belgian Patent No. 873198), ammonium(3-amino-3-carboxypropyl)methylphosphinate ("glufosinate"--see U. S. Patent 4,168,863), (2-amino-4-methylphosphinobutyryl)alanylalanine (2-amino-4-[(hydroxy)(methyl)phosphinyl]butyryl alanylalanine "bialaphos"--U. S. Patent No. 4,309,208) and 1,1′-dimethyl-4,4′-bipyridinium dichloride ("paraquat"--see Dutch Patent No. 6603346) are known as non-selective herbicides for foliar treatment. These known herbicidal compounds, particularly glyphosate, glufosinate and bialaphos, require high dosages for effective treatment, and therefore high weed control costs.

Paraquat has very strong acute toxicity, and its application requires meticulous care and measures for preventing accidents. However, accidents have frequently occurred previously owing to mishandling and misuse of paraquat. It is desired therefore to reduce its effective dosage. Furthermore, paraquat has a narrow herbicidal spectrum and an inferior effect on polygonaceous weeds which are important weeds. In addition, the period of controlling weeds by paraquat is very short, and the number of its applications must be increased. This raises a problem of residual buildup in the soil and a problem of increased weed control costs.

The present inventors made extensive investigations in order to solve the problems associated with the above conventional herbicides, glyphosate, glufosinate, bialaphos and paraquat. These investigations have led to the discovery that when these herbicidal compounds are combined with a certain novel herbicidally active 2-nitro-5-(substituted pyridyloxy)benzohydroximic acid derivative developed by the assignee's company of the present application, the effective dose of the known herbicidal compounds can be drastically reduced, and the combinations exhibit an excellent synergistic herbicidal effect against a very broad range of weeds.

In particular, when the above novel compound is used in combination with paraquat, the effective dosage of paraquat can be drastically reduced, and the above accidents can be obviated. The combined herbicide shows strong activity on polygonaceous weeds which have been difficult to control by paraquat alone. In addition, it has been found that the period of controlling weeds by the combined herbicide can be greatly decreased as compared with paraquat alone.

Thus, according to this invention, there is provided a herbicidal composition comprising

(A) a 2-nitro-5-(substituted pyridyloxy)benzohydroximic acid derivative represented by the following formula

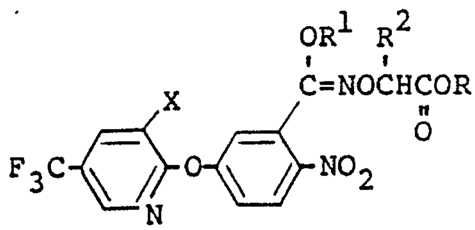

(I)

wherein R and $R^1$, independently from each other, represent a lower alkyl group, $R^2$ represents a hydrogen atom or a lower alkyl group, and X represents a hydrogen or halogen atom [to be referred to as component (A)], and

(B) at least one compound selected from the group consisting of N-(phosphonomethyl)glycine, ammonium(3-amino-3-carboxypropyl)methylphosphinate, (2-amino-4-methylphosphinobutyryl)alanylalanine and 1,1′-dimethyl-4,4′-bipyridinium dichloride [to be referred to as component (B)].

The herbicidal composition provided by this invention will be described below in more detail.

The 2-nitro-5-(substituted pyridyloxy)benzohydroximic acid derivative used as component (A) in the herbicidal composition of this invention is a novel compound not described in prior publications. Details of its properties and herbicidal activity and the method of its production are disclosed in U. S. Patent Application Serial No. 220,068 filed on July 14, 1988 and European Patent Application No. 88110942.5 (European Patent Publication No. 299,382 published on January 18, 1989).

In the compound of formula (I), the "halogen" atom may be, for example, fluorine, chlorine, bromine or iodine. Fluorine and chlorine, especially the latter, are preferred.

The "lower alkyl group" may be a linear or branched alkyl group having up to 6 carbon atoms, such as methyl, ethyl, n-propyl, iso-propyl, n-butyl, sec-butyl, iso-butyl, tert-butyl, n-amyl, iso-amyl or n-hexyl.

In the compound of formula (I) provided by this invention, $R^1$ is preferably a methyl group, and $R^2$ is preferably a hydrogen atom or a methyl group, particularly the former. Conveniently, R is a methyl or ethyl group.

Compounds of formula (I) in which R represents a lower alkyl group, $R^1$ represents a methyl group, $R^2$ represents hydrogen atom or a methyl group, and X represents a hydrogen, fluorine or chlorine atom are preferred as component (A). More preferred are compounds of formula (I) in which R is a lower alkyl group, especially a methyl or ethyl group, $R^1$ is a methyl group, $R^2$ is a hydrogen atom, and X is a chlorine atom.

Especially preferred as the compound of formula (I) provided by this invention are methyl O-methoxycarbonylmethyl-5-(3-chloro-5-trifluoromethyl-2-pyridyloxy)-2-nitrobenzohydroximate and methyl O-ethoxycarbonylmethyl-5-(3-chloro-5-trifluoromethyl-2-pyridyloxy)-2-nitrobenzohydroximate, the latter being preferred above all.

The compound of formula (I) has an asymmetric carbon atom, and may exist as a dextrorotatory, levorotatory or racemic form. The compound of formula (I) includes two stereoisomers represented by formulae (I-a) and (I-b) [syn-type and anti-type]. It should be understood that formula (I) encompasses both of these stereoisomers.

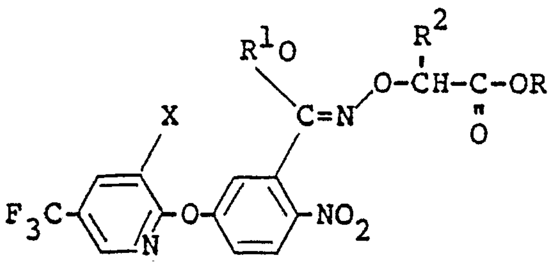

(I-a)

(syn-type)

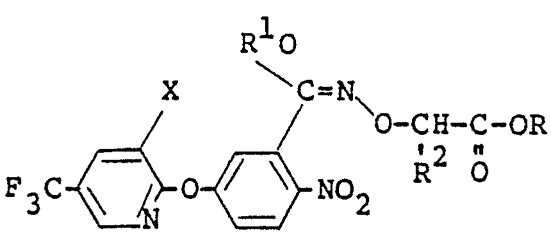

(I-b)

(anti-type)

There is no great difference in herbicidal activity between the two stereoisomers, but the syn-type stereoisomer is generally preferred from the standpoint of mutagenicity.

The compound of this invention can be produced by a process shown by the following reaction scheme A.

## Reaction Scheme A

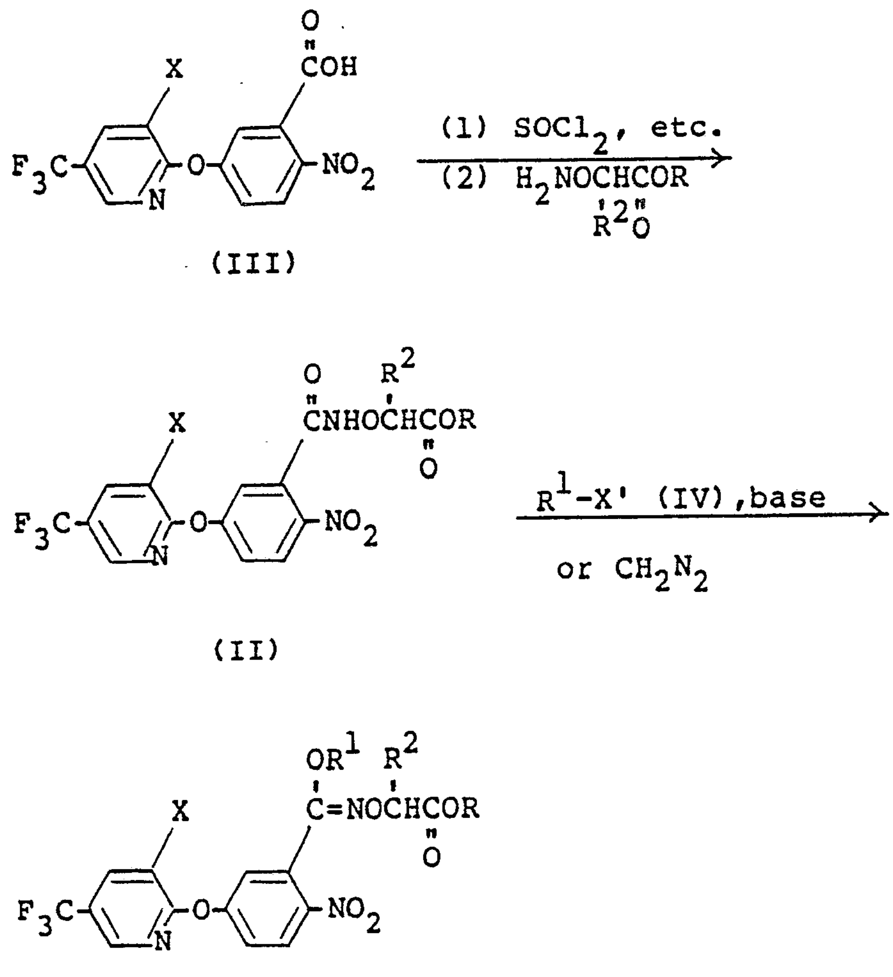


(III)

(II)

(I)

In the above reaction scheme, X, $R^1$, $R^2$ and R are as defined in formula (I). X′ represents a halogen atom or the group-$OSO_2R'$ in which R′ represents a substituted or unsubstituted alkyl, phenyl or alkoxy group.

According to this embodiment, the hydroxamic acid derivative (II) can be obtained by converting the 2-nitro-5-(substituted pyridyloxy)benzoic acid (III) to an acid chloride with, for example, thionyl chloride, and reacting the acid chloride with an O-substituted hydroxylamine, for example in an organic solvent in the presence of a base.

In the reaction, the amount of thionyl chloride used may be properly changed. For example, it may be used in an amount of 1 to 10 equivalents to the compound of formula (III). The reaction temperature may also be properly chosen, and is, for example, room temperature to 80 °C. In the reaction of the acid chloride with the O-substituted hydroxylamine, the amount of the O-substituted hydroxylamine used may be properly chosen, for example, 1 to 3 equivalents, preferably 1 to 1.5 equivalents. The amount of the base can also be properly selected, and is, for example, 1 to 3 equivalents, preferably 1 to 1.5 equivalents. The reaction temperature at this time may be, for example, -10 to 100 °C, preferably 0 °C to room temperature.

Examples of the solvent used include aromatic hydrocarbons such as benzene and toluene, ethers such as tetrahydrofuran and dioxane, acetone, acetonitrile, dimethyl formamide and dimethyl sulfoxide. A mixture of such an organic solvent with water may also be used. Examples of the base are pyridine, triethylamine, sodium carbonate, potassium carbonate and sodium hydrogen carbonate.

The resulting O-substituted-2-nitro-5-(substituted pyridyloxy)benzohydroxamic acid derivative (II) is reacted with an alkylating agent (IV) or $CH_2N_2$ in an organic solvent in the presence of absence of a base to produce the hydroximic acid derivative (I) used in the herbicidal composition of this invention.

The reaction may be carried out by using 1 to 3 molar equivalents, preferably 1 to 1.5 molar equivalents, of the compound (IV) or $CH_2N_2$ with respect to the compound (II) at a temperature of 0 °C (under cooling with ice) to the refluxing temperature of the solvent, preferably room temperature to about 80 °C, for a period of 0.5 to 20 hours. Examples of the solvent used in this reaction are alcohols such as methanol and ethanol, aromatic hydrocarbons such as benzene and toluene, ethers such as diethyl ether, tetrahydrofuran and dioxane, acetone, acetonitrile, dimethyl formamide, and dimethyl sulfoxide. A mixture of such an organic solvent and water may also be used.

Examples of the base used in the above reaction include pyridine, triethylamine, sodium hydroxide, potassium hydroxide, sodium carbonate, potassium carbonate, sodium ethylate, and sodium hydride. An acid binder may be used in an amount of, for example, 1 to 3 equivalents, preferably 1 to 1.5 equivalents, to the compound (IV). The use of the acid binder is preferred because it leads to good yields. When the reaction is carried out using $CH_2N_2$ as the alkylating agent, no base needs to be used.

When the reaction is carried out in a two-phase system, it is possible to use a phase transfer catalyst, for example a quaternary ammonium salt such as tetramethyl ammonium bromide, tetrabutyl ammonium bromide or benzyl tributyl ammonium bromide and a quaternary phosphonium such as tetraphenyl phosphonium bromide in an amount of, for example, 1 to 50 % by weight, preferably 5 to 30 % by weight.

After the reaction, the compound of formula (I) can be isolated by, for example, pouring the reaction mixture in water, and treating it in a customary manner, for example by extraction with an organic solvent, recrystallization or column chromatography.

The following Examples illustrate the synthesis of the compound of formula (I)

## EXAMPLE 1

Production of methyl O-methoxycarbonylmethyl-5-(3-chloro-5-trifluoromethyl-2-pyridyloxy)-2-nitrobenzohydroximate (compound No. 32):-

36.2 g (0.10 mole) of 3-(3-chloro-5-trifluoromethyl-2-pyridyloxy)-2-nitrobenzoic acid was dissolved in 36.2 ml of thionyl chloride, and the solution was heated under reflux for 1.5 hours. The excess of thionyl chloride was evaporated to give an acid chloride.

A solution of the acid chloride in 150 ml of ether was added dropwise to a solution consisting of 10.5 g (0.1 mole) of methyl aminoxyacetate, 10.1 g (0.1 mole) of triethylamine and 300 ml of dry ether with stirring under ice cooling over the course of about 20 minutes. Thereafter, the mixture was stirred under ice cooling for 30 minutes and then at room temperature for 1.5 hours. The reaction solution was poured into 300 ml of ice water, and extracted with 200 ml of ethyl acetate three times. The organic layers were washed with a saturated aqueous solution of sodium chloride and dried over magnesium sulfate. The desiccant was separated by filtration and the solvent was evaporated. The resulting solid was recrystallized from toluene, to give 37.2 g (yield 82.7 %) of methoxycarbonylmethyl 5-(3-chloro-5-trifluoromethyl-2-pyridyloxy)-2-nitrobenzohydroxamate.

The resulting hydroxamate (37.2 g) was dissolved in a mixed solvent composed of 70 ml of ether and 70 ml of tetrahydrofuran, and with stirring under ice cooling, a solution of diazomethane prepared from 24.8 g (0.241 mole) of N-nitrosomethylurea in 300 ml of ether was added dropwise over about 40 minutes. The mixture was then stirred under ice cooling for about 30 minutes. The excess of diazomethane was decomposed with acetic acid, and the solvent was evaporated. The resulting solid was recrystallized from methanol to give 27.0 g (yield 70.4 %) of the desired compound No. 32 (syn-type). mp. 71.0 to 73.0 °C.

5.0 g of the above compound No. 32 was dissolved in 200 ml of toluene. The solution was bubbled with $N_2$, deaerated, and irradiated with a high-pressure mercury lamp (UVL-1009, made by Rikokagaku Sangyo) for 3 hours. The solvent was evaporated, and the residue was purified by column chromatography (silica gel; n-hexane/ethyl acetate (2:1) to give 3.5 g (yield 70.0 %) of an isomer (anti-type; mp. 84.5-85.5 °C) of compound No. 32.

## EXAMPLE 2

Production of ethyl O-methoxycarbonylmethyl-5-(3-chloro-5-trifluoromethyl-2-pyridyloxy)-2-nitrobenzohydroximate (compound No. 53):-

A suspension composed of 2.25 g (5 millimoles) of the hydroxamate obtained in the same way as in Example 1, 0.83 g (6 millimoles) of anhydrous potassium carbonate and 10 ml of DMF was heated to 65 to 67 °C, and 1.2 g (about 6 millimoles) of ethyl p-toluenesulfonate was added dropwise over the course of about 15 minutes. Then, the mixture was stirred at the same temperature as above for 1 hour. The reaction mixture was poured into about 150 ml of ice water, and extracted with 30 ml of ethyl acetate twice. The organic layers were washed with a saturated aqueous solution of sodium chloride, and then dried over magnesium sulfate. The desiccant was separated by filtration, and the solvent was evaporated. The resulting oily substance was purified by column chromatography (silica gel; n-hexane/ethyl acetate 4:1) to give 1.4 g (yield 58.5 %) of the desired compound No. 53. $n_D^{21.1}$ = 1.5359.

Table 1 shows pyridyl phenyl ether derivatives of formula (I) synthesized by techniques similar to those shown in the above Examples.

Table 1

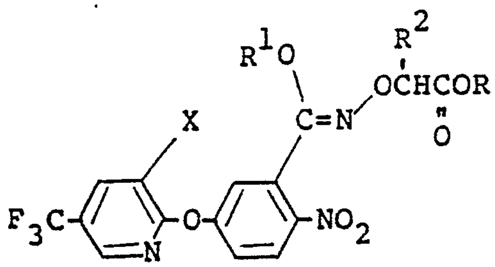

(syn-type)

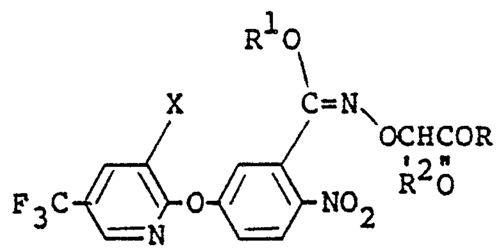

(anti-type)

| Compound No. | X | $R^1$ | $R^2$ | R | Properties |
|---|---|---|---|---|---|
| 1 | H | $CH_3$ | H | $CH_3$ | mp. 81.0-84.0 °C (syn-type) |
| 2 | H | $CH_3$ | H | $C_2H_5$ | |
| 3 | H | $CH_3$ | H | $n-C_3H_7$ | |
| 4 | H | $CH_3$ | H | $i-C_3H_7$ | |
| 5 | H | $CH_3$ | H | $n-C_4H_9$ | mp. 55.0-56.0 °C (anti-type) |
| 6 | H | $CH_3$ | H | $i-C_4H_9$ | |
| 7 | H | $CH_3$ | H | $s-C_4H_9$ | |
| 8 | H | $CH_3$ | H | $t-C_4H_9$ | |
| 9 | H | $CH_3$ | H | $n-C_5H_{11}$ | |
| 10 | H | $CH_3$ | H | $t-C_5H_{11}$ | |
| 11 | H | $CH_3$ | $CH_3$ | $CH_3$ | |
| 12 | H | $CH_3$ | $CH_3$ | $C_2H_5$ | |
| 13 | H | $CH_3$ | $CH_3$ | $n-C_3H_7$ | |
| 14 | H | $CH_3$ | $CH_3$ | $i-C_3H_7$ | |
| 15 | H | $CH_3$ | $CH_3$ | $n-C_4H_9$ | |
| 16 | H | $CH_3$ | CH3 | $t-C_4H_9$ | |
| 17 | H | $CH_3$ | $CH_3$ | $n-C_5H_{11}$ | |
| 18 | H | $CH_3$ | $C_2H_5$ | $CH_3$ | |

- to be continued -

Table 1 (continued)

| Compound No. | X | $R^1$ | $R^2$ | R | Properties |
|---|---|---|---|---|---|
| 19 | H | $CH_3$ | $C_2H_5$ | $C_2H_5$ | |
| 20 | H | $CH_3$ | $C_2H_5$ | $n-C_3H_7$ | |
| 21 | H | $CH_3$ | $C_2H_5$ | $n-C_4H_9$ | |
| 22 | H | $C_2H_5$ | H | $CH_3$ | |
| 23 | H | $C_2H_5$ | H | $C_2H_5$ | |
| 24 | H | $C_2H_5$ | H | $n-C_4H_9$ | |
| 25 | H | $i-C_3H_7$ | H | $CH_3$ | |
| 26 | H | $i-C_3H_7$ | H | $C_2H_5$ | |
| 27 | H | $i-C_3H_7$ | H | $n-C_4H_9$ | |
| 28 | H | $C_2H_5$ | $CH_3$ | $CH_3$ | |
| 29 | H | $C_2H_5$ | $CH_3$ | $n-C_4H_9$ | |
| 30 | H | $i-C_3H_7$ | $CH_3$ | $CH_3$ | |
| 31 | H | $i-C_3H_7$ | $CH_3$ | $n-C_4H_9$ | |
| 32 | Cl | $CH_3$ | H | $CH_3$ | mp. 71.0-73.0 °C (syn-type) mp. 84.5-85.5 °C (anti-type) |
| 33 | Cl | $CH_3$ | H | $C_2H_5$ | mp. 101.5-103.0 °C (syn-type) mp. 58.0-59.5 °C (anti-type) |
| 34 | Cl | $CH_3$ | H | $n-C_3H_7$ | mp. 71.5-73.0 °C (anti-type) |
| 35 | Cl | $CH_3$ | H | $i-C_3H_7$ | mp. 97.5-99.0 °C (anti-type) |
| 36 | Cl | $CH_3$ | H | $n-C_4H_9$ | mp. 84.0-85.0 °C (anti-type) |
| 37 | Cl | $CH_3$ | H | $i-C_4H_9$ | mp. 77.5-79.0 °C (anti-type) |

- to be continued -

Table 1 (continued)

| Compound No. | X | $R^1$ | $R^2$ | R | Properties |
|---|---|---|---|---|---|
| 38 | Cl | $CH_3$ | H | s-$C_4H_9$ | |
| 39 | Cl | $CH_3$ | H | t-$C_4H_9$ | |
| 40 | Cl | $CH_3$ | H | n-$C_5H_{11}$ | mp. 71.5-72.5 °C (anti-type) |
| 41 | Cl | $CH_3$ | H | t-$C_5H_{11}$ | |
| 42 | Cl | $CH_3$ | $CH_3$ | $CH_3$ | $n_D^{20.4}$ 1.5328 (syn-type) |
| 43 | Cl | $CH_3$ | $CH_3$ | $C_2H_5$ | |
| 44 | Cl | $CH_3$ | $CH_3$ | n-$C_3H_7$ | |
| 45 | Cl | $CH_3$ | $CH_3$ | i-$C_3H_7$ | |
| 46 | Cl | $CH_3$ | $CH_3$ | n-$C_4H_9$ | |
| 47 | Cl | $CH_3$ | $CH_3$ | t-$C_4H_9$ | |
| 48 | Cl | $CH_3$ | $CH_3$ | n-$C_5H_{11}$ | |
| 49 | Cl | $CH_3$ | $C_2H_5$ | $CH_3$ | |
| 50 | Cl | $CH_3$ | $C_2H_5$ | $C_2H_5$ | |
| 51 | Cl | $CH_3$ | $C_2H_5$ | n-$C_3H_7$ | |
| 52 | Cl | $CH_3$ | $C_2H_5$ | n-$C_4H_9$ | |
| 53 | Cl | $C_2H_5$ | H | $CH_3$ | $n_D^{21.1}$ 1.5359 (syn-type) |
| 54 | Cl | $C_2H_5$ | H | $C_2H_5$ | |
| 55 | Cl | $C_2H_5$ | H | n-$C_4H_9$ | |
| 56 | Cl | i-$C_3H_7$ | H | $CH_3$ | $n_D^{21.1}$ 1.5336 (syn-type) |
| 57 | Cl | i-$C_3H_7$ | H | $C_2H_5$ | |
| 58 | Cl | i-$C_3H_7$ | H | n-$C_4H_9$ | |

- to be continued -

Table 1 (continued)

| Compound No. | X | $R^1$ | $R^2$ | R | Properties |
|---|---|---|---|---|---|
| 59 | Cl | $C_2H_5$ | $CH_3$ | $CH_3$ | |
| 60 | Cl | $C_2H_5$ | $CH_3$ | $n-C_4H_9$ | |
| 61 | Cl | $i-C_3H_7$ | $CH_3$ | $CH_3$ | |
| 62 | Cl | $i-C_3H_7$ | $CH_3$ | $n-C_4H_9$ | |
| 63 | F | $CH_3$ | H | $CH_3$ | |
| 64 | F | $CH_3$ | H | $C_2H_5$ | |
| 65 | F | $CH_3$ | H | $n-C_3H_7$ | |
| 66 | F | $CH_3$ | H | $i-C_3H_7$ | |
| 67 | F | $CH_3$ | H | $n-C_4H_9$ | |
| 68 | F | $CH_3$ | H | $t-C_4H_9$ | |
| 69 | F | $CH_3$ | H | $s-C_4H_9$ | |

The component (B) used in the herbicidal composition of this invention in combination with the 2-nitro-5-(substituted pyridyloxy)benzohydroximic acid derivative as component (A) is at least one known herbicidal compound selected from the group consisting of glyphosate, glufosinate, bialaphos and paraquat.

The ratio of component (A) to component (B) in the herbicidal composition of this invention is not strictly limited, and may be varied over a broad range depending, for example, upon the situation, the object and the time of applying the final herbicidal composition and also upon which of the four known herbicidal compounds is used. Generally, when glyphosate, glusifosinate and/or bialaphos is used as component (B), component (A) is used in an amount of 0.01 to 10 parts by weight, preferably 0.05 to 2 parts by weight, more preferably 0.1 to 0.5 parts by weight, per part by weight of component (B). When paraquast is used as component (B), the amount of component (A) is conveniently 0.05 to 20 parts by weight, preferably 0.1 to 10 parts by weight, more preferably 0.5 to 2 parts by weight, per part by weight of component (B) (paraquat). These properties of the components (A) and (B) of the composition, the advantages of the invention, particularly the synergistic herbicidal efficacy, can be achieved.

For actual use as a herbicide, the compound of this invention is mixed with agriculturally and horticulturally acceptable carriers or diluents, additives and auxiliary agents by a known method and used in usual formulations of agricultural chemicals such as a dust, granules, a wettable powder, an emulsifiable concentrate, a flowable agent, a soluble powder or a sol.

The carrier or diluent may be any of solid or liquid carriers or diluents generally used in the field of agriculture. Solid carriers include talc or clays typified by kaolinite, montmorillonite and attapulgite; inorganic materials such as mica, pyrophyllite, pumice, vermicullite, gypsum, calcium carbonate, dolomite, diatomaceous earth, magnesium lime, apatite, zeolite, silicic anhydride and synthetic calcium silicate; organic substances of the plant origin such as soybean meal, tobacco powder, walnut powder, wheat flour, wood powder, starch and crystalline cellulose; natural or synthetic polymeric compounds such as coumarone resins, petroleum resins, alkyd resins, polyvinyl chloride, polyalkylene glycols, ketone resins, ester gum, copal gum and dammaqr gum; waxes such as carnauba wax and beeswax; and urea. Suitable liquid carriers or diluents include paraffinic or naphthenic hydrocarbons such as kerosene, mineral oils, spindile oil and white oil; aromatic hydrocarbons such as benzene, toluene, xylene, ethylbenzene, cumene and methylnaphthalene; chlorinated hydrocarbons such as carbon tetrachloride, chloroform, trichloroethylene, monochlorobenzene and o-chlorotoluene; ethers such as dioxane and tetrahydrofuran; ketones such as acetone, methyl ethyl ketone, diisobutyl ketone, cyclohexanone, acetophenone and isophorone; esters such as ethyl acetate, amyl acetate, ethylene glycol acetate, diethylene glycol acetate, dibutyl maleate and diethyl succinate; alcohols such as methanol, n-hexanol, ethylene glycol, diethylene glycol, cyclohexanol and benzyl alcohol; ether alcohols such as ethylene glycol ethyl ether, ethylene glycol phenyl ether, diethylene glycol ethyl ether and diethylene glycol butyl ether; polar solvents such as dimethyl formamide and dimethyl sulfoxide; and water.

Surface-active agents and other adjuvants may be used to emulsify, disperse, wet, spread or bind the active ingredients, adjust its disintegrability, stabilize the active ingredients of the herbicide, improve the flowability of the herbicide, prevent corrosion, or otherwise. The surface-active agents may be nonionic, anionic, cationic and amphoteric. Usually, nonionic and anionic surface-active agents are suitable. Suitable nonionioc surface-active agents include, for example, polyaddition products of ethylene oxide with higher alcohols such as lauryl alcohol, stearyl alcohol and oleyl alcohol; polyaddition products of ethylene oxide with alkylphenols such as isooctylphenol and nonylphenol; polyaddition products of ethylene oxides with alkylnaphthols such as butylnaphthol and octylnaphthol; polyaddion products of ethylene oxide with higher fatty acids such as palmitic acid, stearic acid and oleic acid; polyaddition products of ethylene oxide with mono- or di-alkylphosphoric acids such as stearylphosphoric acid and dilaurylphosphoric acid; poly-addition products of ethylene oxide with amine compounds such as dodecylamine and stearamide; higher fatty acid esters of polyhydric alcohols such as sorbitan, and polyaddition products of ethylene oxide with these esters; and a polyaddition product of ethylene oxide with propylene oxide. Suitable anionic surface-active agents include, for example, alkylsulfuric ester salts such as sodium laurylsulfate and oleyl sulfate amine salt; alkylsulfonic acid salts such as sodium 2-ethylhexenesulfonate; and arylsulfonic acid salts such as sodium isopropylnaphthalenesulfonate, sodium methylenebisnaphthalenesulfonate, sodium ligninsulfonate and sodium dodecylbenzenesulfonate.

To improve the properties of the formulated herbicide and increase its herbicidal effect, the compound of the invention may also be used in combination with other adjuvants including such polymeric compounds as casein, gelatin, albumin, glue, sodium alginate, ligninesulfonates, acid isopropyl phosphate (PAP), gum arabic, carboxymethyl cellulose, methyl cellulose, hydroxyethyl cellulose, polyvinyl alcohol, polyvinyl pyrrolidione and polysaccharides.

The above carriers or diluents and various adjuvants may be used singly or in suitable combinations depending upon the form in which the herbicidal composition is formulated, the place and time of application, etc.

The composition of this invention may, as required, further contain other agricultural chemicals such as fungicices, insecticides, acaricides, other herbicides, plant growth regulating agents, fertilizers and soil conditioners.

The proportion of the active ingredients in the herbicidal composition of this invention in such various formulations vary depending upon the form of the composition. For example, the composition may contain 0.1 to 99 % by weight, preferably 1 to 80 % by weight, of the active ingredients. It should be understood that the "active ingredients", as used herein, denote components (A) and (B) in combination.

The dust usually contains 1 to 25 % by weight of the active ingredients and the remainder being a solid carrier.

The wettable powder usually contains 25 to 90 % by weight of the active ingredients and the remainder being a solid carrier and a dispersing and wetting agent and optionally a protective colloid, a thixotropic agent, a defoamer, etc.

The granules usually contain 1 to 35 % by weight of the active ingredients and the remainder being mostly a solid carrier. The active ingredient is uniformly mixed with the solid carrier, or uniformly fixed to, or adsorbed on, the surface of the solid carrier. The granules have a particle diameter of about 0.2 to 1.5 mm.

The emulsifiable concentrate usually contains 1 to 30 % by weight of the active ingredients, about 5 to 20 % by weight of an emulsifier and the remainder being a liquid carrier and optionally a corrosion inhibitor.

The flowable agent usually contain 5 to 50 % by weight of the active ingredients, 3 to 20 parts by weight of a surface active agent, and the remainder being water. As required, a protective collolidal agent, a thickening agent, a freeze preventing agent, an antiseptic, a defoamer, etc. are added.

The composition of this invention has a broad herbicidal spectrum and very high synergistic herbicidal activity and is useful as a non-selective heribice which exhibits an excellent herbicidal efficacy at low effective dosages.

The composition of this invention can control various weeds growing in non-crop lands and croplands, for example terrestrial narrow-leaved and broad-leaved weeds such as barnyardgrass (Echinochloa crus-galli), large crabgrass (Digitaria sanguinalis), green foxtail (Setaria viridis), goosegrass (Eleusine indica), (Paspalum thunbergii), annual bluegrass (Poa annua), amaranths, polygonums, goosefoot (Chenopodium album), lamb's-quarters (Chenopodium album), velvetleaf (Abutilon theophrasti), morning glories (Ipomoea spp.), cocklebur (Xanthium strumarium), common ragweed (Ambrosia artemisiifolia), common purslane (Portulaca oleracea), devil's beggarticks (Bidens frondosa), sicklepod (Cassia tora), black nightshade (Solanum nigrum), prickly sida (Sida spinosa), creeping woodsorrel (Oxalis corniculata), jimsonweed (Datura stramonium), red clover (Trifolium pratense), chickweed (Stellaria media), Japanese pearlwort (Sagina japonica), shepherd's purse (Capsella bursa-pastoris), bittercress (Cardamine flexuosa), bedstraw (Galium spurium, Galium aparine), poppy (Papaver spp.), scented mayweed (Matricaria chamomilla), Canada thistle (Cirsium arvense), marigold, henbit (Lamium amplexicaule), (Veronica caninotesticulata), annual fleabane (Erigeron annuus), Philadelphia fleabane (Erigeron philadelphicus), horseweed (Erigeron canadensis), and hairy fleabane (Erigeron bonariensis); and aquatic narrowleaved and broadleaved plants such as barnyardgrass (Echinochloa crus-galli), umbrella plant (Cyperus difformis), monochoria (Monochoria vaginalis), falsepimpernel (Lindernia pyxidaria), Mizohakobe (Elatine triandra), Kikashigusa (Rotala indica), bulrush (Typha latifolia), and spikerush (Eleocharis acicularis).

The herbicidal composition of this invention is effective for weed control not only in a non-crop land but also in a crop land before useful crops are sown or transplanted. But this is not limitative, and in a crop land in which useful crops are cultivated, it can be applied by a treating mehtod designed to avoid direct contact with the leaves and stalks of the plants (e.g., diret spray).

The rate of application may be small because the composition of the invention has very high herbicidal activity, and may be properly varied depending upon the types of weeds to be controlled, their growth stage, the place of application, the time of application, weather, etc. Generally, the rate of application, as the total amount of components (A) and (B), is 100 to 10000 g/ha, preferably 200 to 2000 g/ha, more preferably 400 to 1000 g/ha. When glyphosate, glufosinate and/or bialaphos is used as component (B), the suitable effective dose of component (A) is generally 5 to 60 g/ha, preferably 25 to 250 g/ha, more preferably 50 to 200 g/ha. When paraquat is used as component (B), the effective dose of component (A) is generally 5 to 600 g/ha, preferably 25 to 200 g/ha, more preferably 100 to 200 g/ha.

Some examples of the formulation of the herbicidal composition of this invention are given below.

In these examples, all parts are by weight.

## FORMULATION EXAMPLE 1

Emulsifiable concentrate:-

A uniform solution composed of 2 parts of compound No. 37, 3 parts of polyoxyethylene styrylphenyl ether, 2 parts of sodium alkylbenzenesulfonate and 10 parts of xylene was added to 73 parts of a 20 % aqueous solution of glyphosate, and the mixture was stirred for about 20 minutes by a homogenizer (rotating speed 25,000 rpm) to form a uniform emulsion. Then, 10 parts of propylene glycol was added to the emulsion, and the mixture was well shaken to form an emulsifiable concentrate.

## FORMULATION EXAMPLE 2

Emulsifiable concentrate:-

A uniform solution composed of 5 parts of compound No. 35, 3 parts of polyoxyethylene styrylphenyl ether, 2 parts of sodium alkylbenzenesulfonate and 10 parts of xylene was added to 70 parts of a 18.5 % aqueous solution of glufosinate, and the mixture was stirred for about 20 minutes by a homogenizer (rotating speed 25,000 rpm) to form a uniform emulsion. Ten parts of propylene glycol was added, and the mixture was well shaken to form an emulsifiable concentrate.

## FORMULATION EXAMPLE 3

Emulsifiable concentrawte:-

A uniform solution composed of 2 parts of compound No. 53, 3 parts of polyoxyethylene styrylphenyl ether, 2 parts of sodium alkylbenzenesulfonate and 10 parts of xylene was added to 73 parts of a 20 % aqueous solution of bialaphos. The mixture was stirred for about 20 minutes by a homogenizer (rotating speed 25,000 rpm) to form a uniform emulsion. Ten parts of propylene glycol was added and the mixture was well shaken to form an emulsifiable concentrate.

FORMULATION EXAMPLE 4

Wettable powder:-

One hundred parts of a 30 % aqueous solution of glyphosate and 3 parts of alkyl phenyl ether were added dropwise to a mixture of 39 parts of white carbon and 23 parts of clay. They were well mixed and then dried with hot air at 50 °C. Furthermore, 3 parts of compound No. 32 and 2 parts of polyoxyethylene alkylamine were added, and the materials were well mixed with stirring. Subsequently, the mixture was finely pulverized by using a jet mill (supplied by Fujisangyo Kabushiki Kaisha) to form a wettable power.

FORMULATION EXAMPLE 5

Wettable powder:-

A 18.5 % aqueous solution of glufosinate (162 parts) and 3 parts of alkyl phenyl ether were added dropwise to a mixture of 39 parts of white carbon and 20 parts of clay, and they were well mixed and dried with hot air at 50 °C. Furthermore, 6 parts of compound No. 36 and 2 parts of polyoxyethylene alkylamine were added, and the mixture was well stirred and subsequently finely pulverized by a jet mill (supplied by Fujisangyo Kabushiki Kaisha) to form a wettable powder.

FORMULATION EXAMPLE 6

Wettable powder:-

One hundred parts of a 30 % aqueous solution of bialaphos and 3 parts of alkyl phenyl ether were added dropwise to a mixture of 39 parts of white carbon and 33 parts of clay, and they were well mixed. The mixture was then dried with hot air at 50 °C, and further well mixed with stirring with 3 parts of compound No. 33 and 2 parts of polyoxyethylene alkylamine. The mixture was then finely pulverized by a jet mill (supplied by Fujisangyo Kabushiki Kaisha) to form a wettable powder.

FORMULATION EXAMPLE 7

Flowable agent:-

Four parts of compound No. 35, 20 parts of glyphosate, 2 parts of polyoxyethylene nonylphenyl ether, 3

parts of dialkyl sulfosuccinate, 20 parts of a 1 % aqueous solution of carboxymethyl cellulose (CMC), 10 parts of propylene glycol and 41 parts of water were mixed, and pulverized in a wet ball mill to form a flowable agent.

## FORMULATION EXAMPLE 8

Flowable agent:-

Four parts of compound No. 40, 20 parts of glufosinate, 2 parts of polyoxyethylene nonylphenyl ether, 3 parts of dialkyl sulfosuccinate, 20 parts of a 1% aqueous solution of CMC, 10 parts of propylene glycol and 41 parts of water were mixed, and pulverized in a wet ball mill to form a flowable agent.

## FORMULATION EXAMPLE 9

Flowable agent:-

Four parts of compound No. 56, 20 parts of biolaphos, 2 parts of polyoxyethylene nonylphenyl ether, 3 parts of dialkyl sulfosuccinate, 20 parts of a 1% aqueous solution of CMC, 10 parts of propylene glycol and 41 parts of water were mixed, and pulverized in a wet ball mill to form a flowable agent.

## FORMULATION EXAMPLE 10

Emulsifiable concentrate:-

A uniform solution composed of 5 parts of compound No. 32, 3 parts of polyoxyethylene styryl ether, 2 parts of sodium alkylbenzenesulfonate and 10 parts of xylene was added to 70 parts of a 24 % by weight aqueous solution of paraquat. The mixture was stirred for about 20 minutes by a homogenizer (rotating speed 25,000 rpm) to form a uniform emulsion. Ten parts of propylene glycol was added, and the mixture was well shaken to form an emulsifiable concentrate.

## FORMULATION EXAMPLE 11

Wettable powder:

A 24 % aqueous solution of paraquat (83.3 parts) and 3 parts of alkyl phenyl ether were added dropwise to a mixture of 26 parts of white carbon and 39 parts of clay, and they were well mixed. The resulting mixture was dried with hot air at 50 °C, and further well stirred with 1 part of compound No. 33 and 2 parts of polyoxyethylene alkylamine. The mixture was subsequently finely pulverized by a jet mill (supplied by Fujisangyo Kabushiki Kaisha) to form a wattable powder.

## FORMULATION EXAMPLE 12

Flowable agent:-

Five parts of compound No. 35, 20 parts of paraquat, 2 parts of polyoxyethylene nonylphenyl ether, 3 parts of dialkyl sulfosuccinate, 20 parts of 1% CMC, 10 parts of propylene glycol and 40 parts of water were mixed and pulverized by a wet ball mill to form a flowable agent.

The following Test Examples demonstrate the excellent herbicidal activity of the compositions provided by this invention.

## TEST EXAMPLE 1

Foliar treatment:-

Upland farm soil was filled in square pots (30 x 30 x 8 cm). Seeds of various weeds indicated in Table 2 were sown in fixed amounts, and grown in a greenhouse until the plants grew to the 3- to 6-leaf stage.

Each of the test compositions prepared as in Formulation Examples 4 to 6 was diluted in water containing 0.25 % of "Surfactant WK" (a product of Kao Co., Ltd.) as a spreader so that the amount of the active ingredients [components (A) and (B)] was as shown in Table 2. The diluted chemical was applied uniformly to the foliage of the plants in an amount corresponding to 500 liters/ha.

Twenty-one days after the application, the herbicidal effect on the weeds was rated on the following standards, and the results are shown in Table 2.

| Rating Standards (11 ratings) | | |
|---|---|---|
| Rating | Herbicidal effect (herbicidal rate, %, based on a non-treated area) | Phytotoxicity to crops (phytotoxicity rate, %, based on a non-treated area) |
| 0 | 0 | Same as left |
| 1 | over 0 to 10 | |
| 2 | over 10 to 20 | |
| 3 | over 20 to 30 | |
| 4 | over 30 to 40 | |
| 5 | over 40 to 50 | |
| 6 | over 50 to 60 | |
| 7 | over 60 to 70 | |
| 8 | over 70 to 80 | |
| 9 | over 80 to 90 | |
| 10 | over 90 to 100 (withered) | |

Table 2

| Test composition | Amount of the active ingredient (gai/ha) component (A) + (B) | Weed a | b | c | d | e | f |
|---|---|---|---|---|---|---|---|
| No. 32 (syn) + glyphosate | 12.5 + 125 | 10 | 10 | 8 | 10 | 9 | 10 |
| | 25 + 250 | 10 | 10 | 10 | 10 | 10 | 10 |
| No. 32 (anti) + glyphosate | 12.5 + 125 | 10 | 10 | 9 | 10 | 10 | 10 |
| | 25 + 250 | 10 | 10 | 10 | 10 | 10 | 10 |
| No. 33 (syn) + glyphosate | 12.5 + 125 | 10 | 10 | 8 | 10 | 10 | 10 |
| | 25 + 250 | 10 | 10 | 10 | 10 | 10 | 10 |
| No. 33 (anti) + glyphosate | 12.5 + 125 | 10 | 10 | 9 | 10 | 10 | 10 |
| | 25 + 250 | 10 | 10 | 10 | 10 | 10 | 10 |
| No. 32 (syn) + glufosinate | 12.5 + 62.5 | 10 | 10 | 8 | 9 | 10 | 10 |
| | 25 + 125 | 10 | 10 | 10 | 10 | 10 | 10 |
| No. 32 (anti) + glufosinate | 12.5 + 62.5 | 10 | 10 | 8 | 10 | 9 | 10 |
| | 25 + 125 | 10 | 10 | 10 | 10 | 10 | 10 |
| No. 33 (syn) + glufosinate | 12.5 + 62.5 | 10 | 10 | 9 | 10 | 10 | 10 |
| | 25 + 125 | 10 | 10 | 10 | 10 | 10 | 10 |
| No. 33 (anti) + glufosinate | 12.5 + 62.5 | 10 | 10 | 9 | 10 | 10 | 10 |
| | 25 + 125 | 10 | 10 | 10 | 10 | 10 | 10 |

Table 2 (continued)

| Test composition | Amount of the active ingredient (gai/ha) | Weed | | | | | |
|---|---|---|---|---|---|---|---|
| | component (A) + (B) | a | b | c | d | e | f |
| No. 32 (syn) + bialaphos | 12.5 + 125 | 10 | 10 | 8 | 9 | 9 | 10 |
| | 25 + 250 | 10 | 10 | 10 | 10 | 10 | 10 |
| No. 32 (anti) + bialaphos | 12.5 + 125 | 10 | 10 | 9 | 10 | 10 | 10 |
| | 25 + 250 | 10 | 10 | 10 | 10 | 10 | 10 |
| No. 33 (syn) + bialaphos | 12.5 + 125 | 10 | 10 | 8 | 10 | 9 | 10 |
| | 25 + 250 | 10 | 10 | 10 | 10 | 10 | 10 |
| No. 33 (anti) + bialaphos | 12.5 + 125 | 10 | 10 | 9 | 10 | 10 | 9 |
| | 25 + 250 | 10 | 10 | 10 | 10 | 10 | 10 |
| glyphosate alone | 125 | 3 | 2 | 2 | 2 | 2 | 4 |
| | 250 | 6 | 4 | 4 | 5 | 4 | 5 |
| glufosinate alone | 62.5 | 3 | 1 | 2 | 2 | 1 | 2 |
| | 125 | 5 | 2 | 3 | 4 | 4 | 3 |
| bialaphos alone | 125 | 3 | 2 | 3 | 1 | 2 | 3 |
| | 250 | 4 | 3 | 5 | 4 | 5 | 5 |

The weeds in the above and subsequent tables are represented by the following letters a to f.

a: pale smartweed
B: green amaranth
C: morning glory
d: barnyard grass
e: fingergrass
f: giant foxtail

TEST EXAMPLE 2

Foliar treatment:-

A field where fingergrass (about 40 cm tall), giant foxtail (about 40 cm tall) and pale smartweed (about 50 cm tall) were uniformly growing was divided into test areas (1 m x 2 m).

A wettable powder prepared in accordance with Formulation Example 11 was diluted with water containing 0.25 % of "Surfacant WK" (produced by Kao Co., Ltd.) so that the amount of the active ingredients was as shown in Table 3, and sprayed uniformly to the stalks and leaves of the plants at a rate of 1000 liters/ha.

One week, 2 weeks, 4 weeks and 8 weeks after the spraying, the herbicidal effects of the compositions were rated on the above standards, and the results are shown in Table 3.

Table 3

| Test composition | Amount of the active ingredients (gai/ha) | One week after the treatment | | | Two weeks after the treatment | | | Four weeks after the treatment | | | Eight weeks after the treatment | | |
|---|---|---|---|---|---|---|---|---|---|---|---|---|---|
| | component (A) + (B) | Weed | | | Weed | | | Weed | | | Weed | | |
| | | e | f | a | e | f | a | e | f | a | e | f | a |
| No. 32 (syn) + | 200 + 400 | 10 | 10 | 9 | 10 | 10 | 10 | 10 | 10 | 10 | 10 | 10 | 10 |
| paraquat | 100 + 200 | 9 | 9 | 8 | 9 | 9 | 9 | 9 | 9 | 7 | 5 | 5 | 4 |
| No. 32 (syn) + | 200 + 400 | 10 | 10 | 9 | 10 | 10 | 10 | 10 | 10 | 10 | 10 | 10 | 10 |
| paraquat | 100 + 200 | 9 | 10 | 7 | 9 | 10 | 9 | 9 | 10 | 8 | 8 | 10 | 7 |
| paraquat | 800 | 10 | 10 | 3 | 9 | 10 | 0 | 6 | 8 | 0 | 2 | 4 | 0 |
| alone | 400 | 7 | 5 | 0 | 8 | 2 | 0 | 4 | 0 | 0 | 0 | 0 | 0 |

## Claims

1. A herbicidal composition comprising

(A) a 2-nitro-5-(substituted pyridyloxy)benzohydroxymic acid derivative represented by the following formula

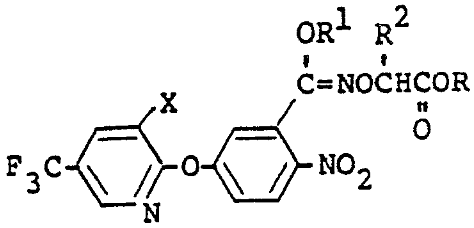

(I)

wherein R and $R^1$, independently from each other, represent a lower alkyl group, $R^2$ represents a hydrogen atom or a lower alkyl group, and X represents a hydrogen or halogen atom, and

(B) at least one compound selected from the group consisting of N-(phosphonomethyl)glycine, ammonium(3-amino-3-carboxypropyl)methylphosphinate, (2-amino-4-methylphosphinobutyryl)alanylalanine and 1,1′-dimethyl-4,4′-bipyridinium dichloride.

2. The herbicidal composition of claim 1 in which component (A) is a compound of formula (I) in which R represetns a lower alkyl group, $R^1$ represents a methyl group, $R^2$ represents a hydrogen atom or a methyl group, and X represents a hydrogen, fluorine or chlorine atom.

3. The herbicidal composition of claim 1 in which component (A) is a compound of formula (I) in which R represents a lower alkyl group, particularly a methyl or ethyl group, $R^1$ represents a methyl group, $R^2$ represents a hydrogen atom, and X represents a chlorine atom.

4. The herbicidal composition of claim 1 in which component (A) is methyl O-methoxycarbonylmethyl 5-(3-chloro-5-trifluorometyl-2-pyridyloxy)-2-nitrobenzohydroximate or methyl O-ethoxycarbonylmethyl 5-(3-chloro-5-trifluoromethyl-2-pyridyloxy)-2-nitrobenzylhydroximate.

5. The herbicidal composition of claim 1 in which component (A) is methyl O-ethoxycarbonylmethyl 5-(3-chloro-5-trifluoromethyl-2-pyridyloxy)-2-nitrobenzyl hydroximate.

6. The herbicidal composition of claim 1 in which component (B) is N-phosphonomethylglycine, ammonium(3-amino-3-carboxypropyl)methylphosphinate or (2-amino-4-methylphosphinobutyryl)-alanylalanine.

7. The herbicidal composition of claim 6 in which the proportion of component (A) is 0.01-10 parts by weight per part by weight of component (B).

8. The herbicidal composition of claim 6 in which the proportion of component (A) is 0.05-2 parts by weight per part by weight of component (B).

9. The herbicidal composition of claim 1 in which component (B) is 1,1′-dimethyl-4,4′-bipyridinium dichloride.

10. The herbicidal composition of claim 9 in which the proportion of component (A) is 0.05-20 parts by weight per part by weight of component (B).

11. The herbicidal composition of claim 9 in which the proportion of component (A) is 0.1-10 parts by weight per part by weight of component (B).

12. A method of controlling weeds which comprises applying a herbicidal composition set forth in claim 1 to the locus where the weeds are to be controlled, or to the weeds to be controlled.